(19) Europäisches Patentamt
European Patent Office
Office européen des brevets

(11) Numéro de publication: **0 484 199 B1**

(12) # FASCICULE DE BREVET EUROPEEN

(45) Date de publication de fascicule du brevet: **21.12.94**

(51) Int. Cl.$^5$: **A61K 7/48**, A61K 7/42, A61K 31/70

(21) Numéro de dépôt: **91402808.9**

(22) Date de dépôt: **22.10.91**

(54) **Utilisation cosmétique d'une composition ayant une activité anti-érythémale.**

(30) Priorité: **30.10.90 FR 9013437**

(43) Date de publication de la demande:
**06.05.92 Bulletin 92/19**

(45) Mention de la délivrance du brevet:
**21.12.94 Bulletin 94/51**

(84) Etats contractants désignés:
**AT BE CH DE DK ES FR GB GR IT LI NL SE**

(56) Documents cités:
**EP-A- 0 256 472**
**WO-A-87/06499**

**PATENT ABSTRACTS OF JAPAN, vol. 14, no. 453 (C-764)[4396], 28 septembre 1990;& JP-A-02 178 233 (TERUMO CORP.) 11-07-1990**

**S.T.N. SERVEUR DE BASES DE DONNEES, Karlsruhe, DE, FICHIER CHEMICAL ABSTRACTS,vol. 96, no. 16, résumé no. 129592F, Columbus, Ohio, US; & JP-A-7757931 (ICHIMARU CO., LTD) 19-05-1977**

**STN SERVEUR DE BASES DE DONNEES, Karlsruhe, DE, FICHIER MEDLINE, AN=86305576;M.L. CUNNINGHAM et al.: "Superoxide anion is generated from cellular metabolites by solar radiation and its components", & J. FREE RADIC. BIOL.MED., (1985) 1 (5-6) 381-5**

(73) Titulaire: **L'OREAL**
**14, Rue Royale**
**F-75008 Paris (FR)**

(72) Inventeur: **Giacomoni, Paolo**
**18, boulevard Sadi Carnot**
**F-95880 Enghien-les-Bains (FR)**
Inventeur: **Morancais, Jean-Luc**
**11, rue Georges Brassens**
**F-77330 Ozoir-la-Ferriere (FR)**
Inventeur: **Lety, Alain**
**9, rue de Metz**
**F-77400 Lagny S/Marne (FR)**

(74) Mandataire: **Peuscet, Jacques et al**
**Cabinet Peuscet**
**68, rue d'Hauteville**
**F-75010 Paris (FR)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services
(3.10/3.09/3.3.3)

## Description

L'invention concerne une utilisation cosmétique par application topique d'une composition ayant une activité anti-érythémale susceptible de protéger la peau des effets nocifs du rayonnement solaire.

On sait que le rayonnement solaire est susceptible de créer sur la peau, en cas d'irradiation trop prolongée ou trop intense, un érythème qui, non seulement, peut être douloureux mais aussi, dans certains cas, peut aller jusqu'à l'état de brûlure. Il est donc bien connu de chercher à protéger la peau, par voie exogène, pour réduire l'érythème solaire au moyen d'une composition cosmétique à application topique, soit en pré-application, soit en post-application par rapport à l'exposition au rayonnement.

Les compositions à effet anti-érythémal sont, à ce jour, réalisées par la mise en oeuvre de l'un ou l'autre de deux principes généraux.

Selon un premier principe, l'application topique de la composition permet de mettre en place sur la peau, des filtres absorbeurs d'ultraviolets A et B ; malheureusement les composés chimiques qui ont des propriétés de filtre n'ont, pour la plupart d'entre eux, qu'une efficacité limitée dans les proportions où ils sont généralement utilisés ; on obtient donc des compositions ayant des indices de protection plus ou moins élevés selon la quantité de filtre mise en oeuvre.

Selon un deuxième principe, au lieu de constituer une barrière externe, on utilise des agents anti-inflammatoires qui traversent la peau et réduisent, au niveau cellulaire, l'effet érythémal des rayonnements ultraviolets ; malheureusement, l'utilisation des agents anti-inflammatoires comporte un certain nombre d'inconvénients. Parmi les agents anti-inflammatoires susceptibles d'être utilisés, on peut citer trois catégories :

a) en premier lieu, les agents anti-inflammatoires non stéroïdiens, tels que l'indométacine et la phénylbutazone ; ces agents, en application topique, provoquent une forte desquamation; s'il y a une pénétration suffisante et un passage dans le système général, ils provoquent des troubles de l'appareil digestif, une altération de la croissance des leucocytes et ils interfèrent avec un certain nombre de médicaments ;

b) en deuxième lieu, les corticoïdes tels que la prednisone, la dexaméthasone ou la cortisone ; ces agents ont malheureusement des effets pléiotropiques : ils font augmenter la teneur en glucose dans le sang, diminuent la réponse immunitaire, augmentent le renouvellement du tissu osseux et provoquent une rétention hydrosodée ;

c) en troisième lieu, les dérivés de l'acide salicylique ; malheureusement ces agents interfèrent avec un certain nombre de médicaments et ralentissent la coagulation.

Il apparaît donc qu'à ce jour, l'utilisation des agents anti-inflammatoires dans des compositions à application topique visant à réduire, au niveau cellulaire, l'effet érythèmal des rayonnements ultraviolets peut difficilement être envisagée sur le plan cosmétique compte tenu des différents inconvénients ci-dessus mentionnés.

La présente invention a pour but de proposer, pour obtenir un effet anti-érythémal, l'utilisation cosmétique d'un composé qui ne présente pas les inconvénients des agents anti-inflammatoires susmentionnés, notamment en raison du fait qu'il s'agit d'un composé présent dans les cellules de l'épiderme à raison de 0,1% du poids sec de celle-ci : le composé en cause est le nicotinamide-adénosine-dinucléotide sous sa forme non-estérifiée (NAD) ou sous sa forme estérifiée par un groupe phosphate (NADP).

Il a déjà été indiqué, dans la demande de brevet européen 256 472, que l'on pouvait utiliser le NAD ou le NADP dans des compositions cosmétiques pour activer les cellules du derme afin d'obtenir une amélioration de l'élasticité de la peau et de sa douceur, une réduction des rides et, de façon générale, un effet anti-vieillissement sur la peau. Selon l'invention, on a maintenant découvert que l'application exogène de NAD ou de NADP permettait de réduire l'érythème solaire de façon efficace sans apparition d'effets secondaires gênants.

Il était connu que le NAD ou le NADP était impliqué dans les mécanismes d'oxydo-réduction qui accompagnent plusieurs processus biochimiques et, en particulier, la glycolyse ; il a été montré que l'effet léthal des radiations ionisantes est du à l'arrêt de la glycolyse et que cet arrêt est causé par la diminution de la quantité de NAD intracellulaire. Il a été montré également qu'une irradiation par un rayonnement ultraviolet entraîne une diminution de la teneur en NAD dans les cellules de l'épiderme (Balard et Giacomoni, Mutation Research, 219 (1989), pages 71-79). Néanmoins, rien ne permettait à l'homme de métier de supposer que l'application topique de NAD ou de NADP était susceptible d'avoir un effet significatif pour la réduction de l'érythème causé par le rayonnement solaire, que ce soit en pré- ou en post-application par rapport à l'exposition au dit rayonnement.

On a également constaté, selon l'invention, que l'effet anti-érythémal susmentionné se manifeste quelle que soit la nature du véhicule de la composition cosmétique, c'est-à-dire qu'il s'agisse d'une solution

aqueuse d'une émulsion ou d'une encapsulation dans des vésicules lipidiques à structure lamellaire. On a constaté également que l'effet anti-érythémal est d'autant plus marqué que la quantité de NAD ou de NADP appliquée est plus importante.

La présente invention a, en conséquence, pour objet l'utilisation cosmétique du nicotinamide-adénosine-dinucléotide (NAD) et/ou du nicotinamide-adénosine-dinucléotide-phosphate (NADP) en application topique pour réduire l'érythème correspondant à une irradiation solaire.

On peut avantageusement prévoir que le NAD et/ou le NADP est (sont) dans une solution aqueuse encapsulée dans des vésicules à structure lipidique lamellaire ; dans ce cas, les vésicules peuvent être définies par des feuillets lipidiques obtenus à partir de lipides ioniqués ou non-ioniques. Néanmoins, le véhicule de la composition peut également être une solution aqueuse ou une émulsion.

Dans l'utilisation selon l'invention, le NAD et/ou le NADP peut (ou peuvent) être appliqué(s) sur la peau avant ou après irradiation de celle-ci ; on préfère que cette application soit réalisée à raison de 10 à 20 mg/cm$^2$. L'application peut avantageusement être faite en étalant sur la peau une composition contenant de 0,05 à 1 % en poids et, de préférence, de 0,2 à 1 % en poids, de NAD ou de NADP par rapport au poids total de la composition. De préférence on utilise une solution aqueuse de NAD dans des vésicules de lipides non-ioniques.

L'invention a également pour objet une composition cosmétique destinée à la mise en oeuvre de l'utilisation selon l'invention telle que ci-dessus définie. Une telle composition peut, avantageusement, contenir, dans un véhicule cosmétiquement acceptable, de 0,05 à 1 % en poids de NAD ou de NADP par rapport au poids total de la composition ; dans cette composition, le NAD et/ou le NADP peut (ou peuvent) être dans une solution aqueuse encapsulée dans des vésicules à structure lipidique lamellaire, ces vésicules étant définies par des feuillets lipidiques obtenus à partir de lipides ioniques ou non-ioniques. Cependant, dans cette composition, le véhicule du NAD et/ou du NADP peut aussi être une solution aqueuse ou une émulsion. Lorsque le NAD ou le NADP est encapsulé dans des vésicules à structure lipidique lamellaire constituées par des lipides amphiphiles non-ioniques, ceux-ci peuvent avantageusement, être choisis dans le groupe formé par

(1) les éthers de polyglycérol, linéaires ou ramifiés, de formule :

$$R\,O\text{-}\!\!\left[C_3H_5(OH)O\right]_{\bar{n}}\text{-}H$$

dans laquelle :

- $-C_3H_5(OH)O$ est représenté par les structures suivantes prises en mélange ou séparément :
  $-CH_2CHOHCH_2O-$ ;

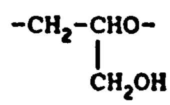 ; 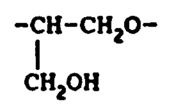 ;

- $\bar{n}$ est une valeur statistique moyenne comprise entre 1 et 6 ;
- R représente :
  (a) une chaîne aliphatique, linéaire ou ramifiée, saturée ou insaturée, contenant de 12 à 30 atomes de carbone ; ou des radicaux hydrocarbonés des alcools de lanoline ;
  (b) un reste $R^1CO$, où $R^1$ est un radical aliphatique, linéaire ou ramifié, en $C_{11}$-$C_{17}$ ;
  (c) un reste $R^2$-[$-OC_2H_3(R^3)$-]-, où :
  - $R^2$ peut prendre la signification (a) ou (b) donnée pour R ;
  - $OC_2H_3(R^3)$- est représenté par les structures suivantes, prises en mélange ou séparément :

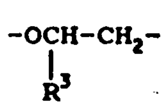 et 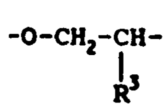

où $R^3$ prend la signification (a) donnée pour R ;

(2) les éthers de polyglycérol, linéaires ou ramifiés, comportant deux chaînes grasses ;

(3) les alcools gras polyoxyéthylénés et les stérols et phytostérols polyoxyéthylénés ;

(4) les éthers de polyols ;

(5) les esters de polyols, oxyéthylénés ou non ;
(6) les glycolipides d'origine naturelle ou synthétique ;
(7) les hydroxyamides représentés par la formule :

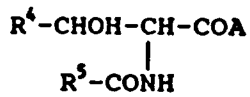

dans laquelle :

- $R^4$ désigne un radical alkyle ou alcényle en $C_7$-$C_{21}$ ;
- $R^5$ désigne un radical hydrocarboné, saturé ou insaturé, en $C_7$-$C_{31}$ ;
- COA désigne un groupement choisi parmi les deux groupements suivants :
  - un reste

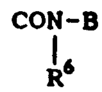

  où :
    - B est un radical dérivé d'amines primaires ou secondaires, mono- ou polyhydroxylées ; et
    - $R^6$ désigne un atome d'hydrogène ou un radical méthyle, éthyle ou hydroxyéthyle ; et
  - un reste -COOZ, où Z représente le reste d'un polyol en $C_3$-$C_7$.

Dans le cas ou le NAD et/ou le NADP est (ou sont) encapsulé(s) dans des vésicules à structure lipidique lamellaire constituées à partir de lipides ioniques, ceux-ci peuvent, avantageusement, être choisis dans le groupe formé par les phospholipides naturels ou de synthèse et les composés anioniques.

Lorsque le NAD et/ou le NADP sont encapsulés dans des vésicules comme ci-dessus indiqué on peut associer aux lipides constitutifs des feuillets des vésicules, au moins un additif choisi dans le groupe formé par :

- les alcools et diols à longue chaîne ;
- les stérols ;
- les amines à longue chaîne et leurs dérivés ammonium-quaternaires ;
- les dihydroxyalkyl-amines ; les amines grasses polyoxyéthylénées ; les esters d'amino-alcools à longue chaîne ; et leurs sels et dérivés ammonium-quaternaires ;
- les esters phosphoriques d'alcools gras ;
- les alkysulfates ;
- certains polypeptides et certaines protéines.

Une composition préférée selon l'invention telle que définie ci-dessus contient du NAD encapsulé dans des vésicules de lipides amphiphiles non-ioniques.

Les exemples ci-après montrent l'efficacité des compositions selon l'invention pour réduire l'érythème solaire en pré ou post-application.

EXEMPLE 1 : Application avant irradiation (comparatif)

Dans cet exemple, on va comparer l'effet en pré-application sur des cobayes d'une irradiation aux ultraviolets dans le cas où l'on utilise soit une dispersion vésiculaire contenant du NAD, soit une dispersion vésiculaire témoin.

a) Préparation de la dispersion vésiculaire témoin (composition A)

On réalise, dans la proportion pondérale 47, 5/47, 5/5, une association de trois lipides à savoir le lipide non-ionique de formule $C_{16}H_{33}OCH_2CHOHCH_2OCH_2CHOHCH_2OH$, de cholestérol et de dicétylphosphate de sodium. Cette association de lipides est obtenue par fusion sous azote à 130 °C. On mélange à poids égaux l'association de lipides ainsi obtenue et un tampon PBS et on laisse l'hydratation se faire à 75 °C pour obtenir une phase lamellaire. On ajoute ensuite dans cette phase lamellaire du tampon PBS (5,25 fois le poids de la phase lamellaire), on disperse les lipides pour obtenir des vésicules (secouage à 50 °C pendant 2 heures) et on soumet la dispersion à l'action des ultrasons.

La dispersion ainsi obtenue est à 8 % en poids de lipides dans le tampon PBS par rapport au poids total de la composition. Le diamètre moyen des vésicules est de 206 ± 4 nm ; le facteur de polydispersité en taille est de 0,31 ± 0,02 ; la fraction volumique des vésicules (c'est-à-dire le volume occupé par les vésicules par rapport à l'ensemble de la dispersion) est de 27 % ; le taux d'encapsulation des vésicules (c'est-à-dire le volume encapsulé dans les vésicules par unité de poids de lipides) est de 2,4 µl/mg.

b) Préparation d'une dispersion vésiculaire contenant le NAD (composition B)

On prépare une solution de NAD dans le tampon PBS à 2 x $10^{-2}$ M.

On prépare une dispersion vésiculaire comme indiqué sous a) en remplaçant le tampon PBS par la solution de NAD préparée comme ci-dessus indiqué. On obtient ainsi une dispersion à 8 % en poids de lipides par rapport au poids total de la composition. Le diamètre moyen des vésicules est de 206 ± 4 nm ; le facteur de polydispersité en taille est de 0,31 ± 0,02; la fraction volumique des vésicules est de 26 % ; le taux d'encapsulation des vésicules est de 2,2 µl/mg.

Les deux compositions vésiculaires préparées comme indiqué sous a) et b) ci-dessus ont donné lieu à des tests comparatifs sur des cobayes albinos de souche Hartley pesant de 350 à 400g. Le dos des animaux est épilé 72 heures avant irradiation. L'irradiation est effectuée avec des tubes générateurs d'UVB de façon à avoir au niveau du dos d'un animal irradié 2 mw/$cm^2$ d'UVB. Sur chaque animal, on délimite deux sites sur chaque flanc, le flanc droit n'étant pas traité. Un masque délimite la zone irradiée. Les produits d'essai sont appliqués par massage digital doux, pendant 30 secondes environ, à raison de 0,1 ml par site (surface traitée = 7 $cm^2$). Pour chaque série d'essais, on utilise un lot de 10 à 15 animaux.

Si l'application avait lieu après irradiation, ce qui n'est pas le cas dans cet exemple mais correspond à l'exemple 2, elle serait réalisée juste après la fin de l'irradiation, puis 30 minutes et 60 minutes après, c'est-à-dire en trois fois.

Une irradiation dure environ une heure trente. Trois heures trente après le début de l'irradiation, les sites traités sont lavés à l'eau tiède et essuyés; une demi-heure après, les érythèmes sont appréciés par un observateur entraîné selon le score suivant :

| SCORE | ERYTHEME |
|---|---|
| 0 | Pas d'érythème |
| 0,5 | Erythème à peine visible |
| 1 | Erythème égal à une dose minimale érythémateuse (MED) |
| 2 | Erythème net |
| 3 | Erythème marqué |
| 4 | Erythème intense |

Si un léger oedème est visible en plus de l'érythème, le score est augmenté d'un point. Le même examen avec évaluation est effectué également 24 heures après le début de l'irradiation.

Les scores visuels des flancs gauches sont comparés, par un test de Wilcoxon pour séries appariées, à ceux obtenus pour les flancs droits uniquement irradiés. Si la différence est significative, un pourcentage d'inhibition P de l'érythème est calculé de la façon suivante :

$$P = 100 \times \frac{SMD - SMG}{SMD}$$

avec

SMD = score moyen correspondant au flanc droit non traité des animaux

SMG = score moyen correspondant au flanc gauche traité des animaux.

Les résultats obtenus ont été consignés dans le tableau I suivant. Les résultats correspondant aux compositions A et B, préparées comme indiqué respectivement aux points a) et b) du présent exemple, ont été comparés aux résultats témoins correspondant à une irradiation sans aucun traitement réalisée dans les mêmes conditions. On constate que la composition B fait apparaître, au bout de 24 heures, un résultat significatif correspondant à un pourcentage d'inhibition de 38 %, alors que la composition vésiculaire A, qui ne contient ni NAD ni NADP, ne donne aucun résultat significatif.

TABLEAU I

| | SMG | | P (%) | | Significativité | |
|---|---|---|---|---|---|---|
| | 4 h | 24 h | 4 h | 24 h | 4 h | 24 h |
| Témoin | 3,40 | 1,88 | | | N.S. | S. |
| Composition B | 3,42 | 1,17 | - | 38 % | | |
| Témoin | 3,58 | 1,44 | | | N.S. | N.S. |
| Composition A | 3,54 | 1,38 | - | - | | |

N.S. = non significatif
S. = significatif

EXEMPLE 2 : Application après irradiation

On prépare une composition A constituée par un tampon PBS.

On prépare une composition B constituée par une solution de NAD à 2 x $10^{-2}$M dans la composition A.

On prépare une composition C comme indiqué pour la composition A de l'exemple 1. Dans ce cas, le diamètre moyen des vésicules obtenues est de 206 ± 3 nm ; le facteur de polydispersité en taille est de 0,26 ± 0,02 ; la fraction volumique des vésicules est de 53 % ; le taux d'encapsulation des vésicules est de 5,6 µl/mg.

On prépare une composition D comme indiqué pour la composition B de l'exemple 1, les vésicules encapsulant le NAD. Dans ce cas, le diamètre moyen des vésicules est de 184 ± 2 nm ; le facteur de polydispersité en taille est de 0,22 ± 0,02 ; la fraction volumique des vésicules est de 40 % ; le taux d'encapsulation des vésicules est de 4 µl/mg.

On compare les résultats obtenus avec les compositions A, B, C, D ci-dessus définies aux résultats obtenus avec des témoins non traités ; le mode d'application des compositions et le mode d'irradiation est celui défini pour l'exemple 1 ; la quantification des résultats est la même que celle définie pour l'exemple 1. Les scores moyens des flancs gauches traités sont donnés dans le tableau II sous le nom SMG ; le pourcentage d'inhibition est calculé lorsque les résultats des traitements sont significatifs par rapport aux témoins. Comme pour l'exemple 1, les scores sont relevés quatre heures et vingt-quatre heures après le début d'irradiation.

D'après les définitions qui ont été données ci-dessus, seules les compositions B et D de cet exemple sont des compositions selon l'invention. Le tableau fait apparaître que les compositions B et D donnent des résultats significatifs vingt-quatre heures après l'irradiation, à savoir respectivement des pourcentages d'inhibition de 41 % et de 62 %. En outre, la composition D donne un résultat significatif quatre heures après le début d'irradiation avec un pourcentage d'inhibition de 20 %. On constate donc que l'effet anti-érythémal du NAD apparaît quel que soit le véhicule mais qu'il est plus marqué lorsque le NAD est encapsulé dans des vésicules.

TABLEAU II

| | SMG | | P (%) | | Significativité | |
|---|---|---|---|---|---|---|
| | 4 h | 24 h | 4 h | 24 h | 4 h | 24 h |
| Témoin | 2.50 | 0.77 | | | S. | S. |
| Composition D | 2.00 | 0.29 | 20 % | 62 % | | |
| Témoin | 2.17 | 0.90 | | | N.S. | N.S. |
| Composition C | 2.42 | 0.69 | - | - | | |
| Témoin | 2.71 | 1.23 | | | N.S. | S. |
| Composition B | 2.31 | 0.73 | - | 41 % | | |
| Témoin | 2.50 | 1.15 | | | N.S. | N.S. |
| Composition A | 2.88 | 1.46 | - | - | | |

N.S. = non significatif S. = significatif

EXEMPLE 3 :

On prépare la formulation suivante :

| | |
|---|---|
| - Lipide amphiphile non ionique de formule $C_{16}H_{33}OCH_2CHOHCH_2OCH_2CHOHCH_2OH$ | 4,75g |
| - Cholestérol | 4,75g |
| - Dicétylphosphate de sodium | 0,5 g |
| - Glycérine | 4,0 g |
| - Parfum | qsp |
| - Conservateur | qsp |
| - Solution de NAD dans le tampon PBS à 2 X $10^{-2}$ M | qsp 100 g |

La composition est préparée comme à l'exemple 1. La glycérine est introduite avant l'hydratation de la phase lipidique. Le parfum et le conservateur sont introduits au moment de la dilution de la phase vésiculaire.

La composition est un fluide de soins ; elle est appliquée après exposition au soleil, à raison de 14 mg/cm$^2$ tout de suite après l'exposition, puis une heure et deux heures après l'exposition. L'intensité de l'érythème solaire est notablement réduit.

EXEMPLE 4 :

On prépare la formulation suivante :

| | |
|---|---|
| - Lipide amphiphile non ionique de formule : $C_{16}H_{33}OCH_2CHOHCH_2OCH_2CHOHCH_2OH$ | 4,75g |
| - Cholestérol | 4,75g |
| - Dicétylphosphate de sodium | 0,5 g |
| - Solution de NAD dans le tampon PBS à 2 X $10^{-2}$ M | qsp 45,0 g |

La dispersion vésiculaire est préparée comme à l'exemple 3. On ajoute à cette dispersion les substances suivantes :

| | |
|---|---|
| - Parfum | 0,2g |
| - Huile de Purcellin | 25,0 g |
| - Mélange d'acides carboxyvinyliques commercialisé sous le nom de "Carbopol 940" | 0,2 g |
| - Triéthanolamine | 0,2g |
| - Solution de NAD dans le tampon PBS à 2 X $10^{-2}$ M | qsp 15,0g |

L'application de cette crème de soins s'effectue comme à l'exemple 3 et donne les mêmes résultats.

EXEMPLE 5 :

On prépare la formulation suivante :

| | |
|---|---|
| - Stéarate de glycérol | 2 g |
| - Tween 60 (monostéarate de sorbitan à 20 moles OE) | 1 g |
| - Alcool cétylique | 0,5 g |
| - Acide stéarique | 1 g |
| - Carbopol 940 | 0,2 g |
| - Perhydrosqualène | 15 g |
| - Fraction liquide de graisse de karité | 10 g |
| - Parfum | 0,1 g |
| - Solution à 2 X $10^{-2}$ M de NAD dans PBS | qsp 100 g |

L'application de cette crème de soins s'effectue comme à l'exemple 3 et donne les mêmes résultats.

## Revendications

1. Utilisation cosmétique du nicotinamide-adénosine-dinucléotide (NAD) et/ou du nicotinamide-adénosine-dinucléotide-phosphate (NADP) en application topique pour réduire l'érythème correspondant à une irradiation solaire.

2. Utilisation selon la revendication 1, caractérisée par le fait que le NAD et/ou le NADP est (ou sont) dans une solution aqueuse encapsulée dans des vésicules à structure lipidique lamellaire.

3. Utilisation selon la revendication 2, caractérisée par le fait que les vésicules sont définies par des feuillets lipidiques obtenus à partir de lipides non-ioniques.

4. Utilisation selon l'une des revendications 1 à 3, caractérisée par le fait que l'on utilise une solution aqueuse de NAD encapsulée dans des vésicules de lipides non-ioniques.

5. Utilisation selon la revendication 2, caractérisée par le fait que les vésicules sont définies par des feuillets lipidiques obtenus à partir de lipides ioniques.

6. Utilisation selon la revendication 1, caractérisée par le fait que le NAD et/ou le NADP est (ou sont) dans une solution aqueuse non-encapsulée.

7. Utilisation selon la revendication 1, caractérisée par le fait que le NAD et/ou le NADP est (ou sont) dans une émulsion.

8. Utilisation selon l'une des revendications 1 à 7, caractérisée par le fait que le NAD et/ou le NADP est (ou sont) appliqué(s) sur la peau avant irradiation de celle-ci.

9. Utilisation selon l'une des revendications 1 à 7, caractérisée par le fait que le NAD et/ou le NADP est (ou sont) appliqué(s) sur la peau après irradiation de celle-ci.

10. Utilisation selon l'une des revendications 1 à 9, caractérisée par le fait que le NAD et/ou le NADP est (ou sont) appliqué (s) sur la peau à raison de 10 à 20 mg/cm$^2$.

11. Utilisation selon l'une des revendications 1 à 10 caractérisée par le fait que le NAD et/ou ou le NADP est (ou sont) appliqué (s) sur la peau par étalement sur celle-ci d'une composition contenant de 0,05 à 1 % en poids de NAD ou de NADP par rapport au poids total de la composition.

12. Composition cosmétique destinée à la mise en oeuvre de l'utilisation selon l'une des revendications 1 à 11 caractérisée par le fait qu'elle contient, dans un véhicule cosmétiquement acceptable, de 0,05 à 1 % en poids de NAD et/ou de NADP par rapport au poids total de la composition.

13. Composition selon la revendication 12, caractérisée par le fait que le NAD et/ou le NADP est (ou sont) dans une solution aqueuse encapsulée dans des vésicules à structure lipidique lamellaire.

14. Composition selon la revendication 13, caractérisée par le fait que les vésicules sont définies par des feuillets lipidiques obtenus à partir de lipides non-ioniques.

15. Composition selon l'une des revendications 12 à 14 caractérisée par le fait que la composition contient du NAD dans une solution aqueuse encapsulée dans des vésicules de lipides non-ioniques.

16. Composition selon la revendication 14 ou 15, caractérisée par le fait que les lipides amphiphiles non-ioniques sont choisis dans le groupeformé par :
    (1) les éthers de polyglycérol, linéaires ou ramifiés, de formule :

$$RO-\!\!\!\left[C_3H_5(OH)O\right]_{\overline{n}}-H$$

dans laquelle :

- $-C_3H_5(OH)O$ est représenté par les structures suivantes prises en mélange ou séparément : $-CH_2CHOHCH_2O-$ ;

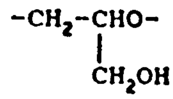 ; 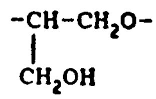 ;

- $\bar{n}$ est une valeur statistique moyenne comprise entre 1 et 6 ;
- R représente :

  (a) une chaîne aliphatique, linéaire ou ramifiée, saturée ou insaturée, contenant de 12 à 30 atomes de carbone ; ou des radicaux hydrocarbonés des alcools de lanoline ;

  (b) un reste $R^1CO$, où $R^1$ est un radical aliphatique, linéaire ou ramifié, en $C_{11}$-$C_{17}$ ;

  (c) un reste $R^2$-[$-OC_2H_3(R^3)$-]-, où :

  - $R^2$ peut prendre la signification (a) ou (b) donnée pour R ;
  - $OC_2H_3(R^3)$- est représenté par les structures suivantes, prises en mélange ou séparément :

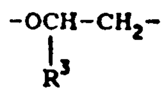 et 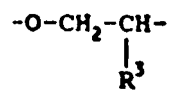

où $R^3$ prend la signification (a) donnée pour R ;

(2) les éthers de polyglycérol, linéaires ou ramifiés, comportant deux chaînes grasses ;

(3) les alcools gras polyoxyéthylénés et les stérols et phytostérols polyoxyéthylénés ;

(4) les éthers de polyols ;

(5) les esters de polyols, oxyéthylénés ou non ;

(6) les glycolipides d'origine naturelle ou synthétique ;

(7) les hydroxyamides représentés par la formule :

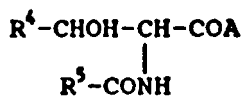

dans laquelle :

- $R^4$ désigne un radical alkyle ou alcényle en $C_7$-$C_{21}$ ;
- $R^5$ désigne un radical hydrocarboné, saturé ou insaturé, en $C_7$-$C_{31}$ ;
- COA désigne un groupement choisi parmi les deux groupements suivants :
  - un reste

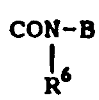

où :

- B est un radical dérivé d'amines primaires ou secondaires, mono- ou polyhydroxylées ; et
- $R^6$ désigne un atome d'hydrogène ou un radical méthyle, éthyle ou hydroxyéthyle ; et

  - un reste $-COOZ$, où Z représente le reste d'un polyol en $C_3$-$C_7$.

**17.** Composition selon la revendication 13, caractérisée par le fait que les vésicules sont définies par des feuillets lipidiques obtenus à partir de lipides ioniques.

**18.** Composition selon la revendication 17, caractérisée par le fait que les lipides amphiphiles ioniques sont choisis dans le groupe formé par les phospholipides naturels ou de synthèse et les composés anioniques.

19. Composition selon l'une des revendications 13 à 18, caractérisée par le fait qu'au(x) lipide(s) constitutif-(s) des feuillets des vésicules est associé au moins un additif choisi dans le groupe formé par
    - les alcools et diols à longue chaîne ;
    - les stérols ;
    - les amines à longue chaîne et leurs dérivés ammonium-quaternaires ;
    - les dihydroxyalkyl-amines ; les amines grasses polyoxyéthylénées ; les esters d'amino-alcools à longue chaîne ; et leurs sels et dérivés ammonium-quaternaires ;
    - les esters phosphoriques d'alcools gras ;
    - les alkysulfates ;
    - certains polypeptides et certaines protéines.

20. Composition selon la revendication 12, caractérisée par le fait que le véhicule de ladite composition est une solution aqueuse.

21. Composition selon la revendication 12, caractérisée par le fait que le véhicule de ladite composition est une émulsion.

**Claims**

1. Cosmetic use of nicotinamide-adenosine dinucleotide (NAB) and/or of nicotinamide-adenosine dinucleotide phosphate (NADP) in topical application for reducing erythema corresponding to solar irradiation.

2. Use according to Claim 1, characterized in that the NAB and/or NADP is (or are) in an aqueous solution encapsulated in vesicles having a lamellar lipid structure.

3. Use according to Claim 2, characterized in that the vesicles are defined by lipid lamellae obtained from nonionic lipids.

4. Use according to one of Claims 1 to 3, characterized in that an aqueous solution of NAD encapsulated in nonionic lipid vesicles is used.

5. Use according to Claim 2, characterized in that the vesicles are defined by lipid lamellae obtained from ionic lipids.

6. Use according to Claim 1, characterized in that the NAD and/or NADP is (or are) in an unencapsulated aqueous solution.

7. Use according to Claim 1, characterized in that the NAD and/or NADP is (or are) in an emulsion.

8. Use according to one of Claims 1 to 7, characterized in that the NAD and/or NADP is (or are) applied to the skin before irradiation of the latter.

9. Use according to one of Claims 1 to 7, characterized in that the NAD and/or NADP is (or are) applied to the skin after irradiation of the latter.

10. Use according to one of Claims 1 to 9, characterized in that the NAD and/or NADP is (or are) applied to the skin in the proportion of 10 to 20 $mg/cm^2$.

11. Use according to one of Claims 1 to 10, characterized in that the NAD and/or NADP is (or are) applied to the skin by spreading on the latter a composition containing from 0.05 to 1% by weight of NAD or of NADP relative to the total weight of the composition.

12. Cosmetic composition intended for implementing the use according to one of Claims 1 to 11, characterized in that it contains, in a cosmetically acceptable vehicle, from 0.05 to 1% by weight of NAD and/or of NADP relative to the total weight of the composition.

**13.** Composition according to Claim 12, characterized in that the NAD and/or NADP is (or are) in an aqueous solution encapsulated in vesicles having a lamellar lipid structure.

**14.** Composition according to Claim 13, characterized in that the vesicles are defined by lipid lamellae obtained from nonionic lipids.

**15.** Composition according to one of Claims 12 to 14, characterized in that the composition contains NAD in an aqueous solution encapsulated in nonionic lipid vesicles.

**16.** Composition according to Claim 14 or 15, characterized in that the nonionic amphiphilic lipids are chosen from the group composed of:

(1) linear or branched polyglycerol ethers of formula:

$$R\,O\text{-}[C_3H_5(OH)O]_{\bar{n}}\text{-}H$$

in which:

- $-C_3H_5(OH)O$ is represented by the following structures, taken mixed or separately: $-CH_2CHOHCH_2O-$;

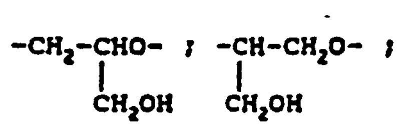

- $\bar{n}$ is an average statistical value between 1 and 6;
- R represents:
  (a) a saturated or unsaturated, linear or branched aliphatic chain containing from 12 to 30 carbon atoms; or hydrocarbon radicals of lanolin alcohols;
  (b) a residue $R^1CO$, where $R^1$ is a linear or branched $C_{11}$-$C_{17}$ aliphatic radical;
  (c) a residue $R^2$-[$-OC_2H_3(R^3)$-]-, where:
    - $R^2$ can take the meaning (a) or (b) given for R;
    - $OC_2H_3(R^3)$- is represented by the following structures, taken mixed or separately:

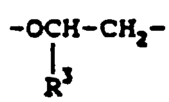

and

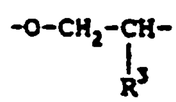

where $R^3$ takes the meaning (a) given for R;

(2) linear or branched polyglycerol ethers containing two fatty chains;
(3) polyoxyethylenated fatty alcohols and polyoxyethylenated sterols and phytosterols;
(4) polyol ethers;
(5) polyol esters, oxyethylenated or otherwise;
(6) glycolipids of natural or synthetic origin;
(7) hydroxyamides represented by the formula:

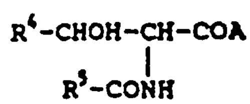

in which:

- $R^4$ denotes a $C_7$-$C_{21}$ alkyl or alkenyl radical;

- $R^5$ denotes a saturated or unsaturated $C_7$-$C_{31}$ hydrocarbon radical;
- COA denotes a group chosen from the following two groups:
  - a residue

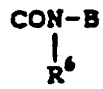

  where:
    - B is a radical derived from mono- or polyhydroxylated primary or secondary amines; and
    - $R^6$ denotes a hydrogen atom or a methyl, ethyl or hydroxyethyl radical; and
  - a residue -COOZ, where Z represents the residue of a $C_3$-$C_7$ polyol.

**17.** Composition according to Claim 13, characterized in that the vesicles are defined by lipid lamellae obtained from ionic lipids.

**18.** Composition according to Claim 17, characterized in that the ionic amphiphilic lipids are chosen from the group composed of natural or synthetic phospholipids and anionic compounds.

**19.** Composition according to one of Claims 13 to 18, characterized in that at least one additive chosen from the group composed of
- long-chain alcohols and diols;
- sterols;
- long-chain amines and their quaternary ammonium derivatives;
- dihydroxyalkylamines; polyoxyethylenated fatty amines; esters of long-chain amino alcohols; and their salts and quaternary ammonium derivatives;
- phosphoric esters of fatty alcohols;
- alkyl sulphates;
- certain polypeptides and certain proteins,

is combined with the lipid(s) constituting the lamellae of the vesicles.

**20.** Composition according to Claim 12, characterized in that the vehicle of the said composition is an aqueous solution.

**21.** Composition according to Claim 12, characterized in that the vehicle of the said composition is an emulsion.

**Patentansprüche**

**1.** Kosmetische Verwendung von Nikotinamid-Adenosin-Dinukleotid (NAD) und/oder Nikotinamid-Adenosin-Dinukleotid-Phosphat (NADP) zur topischen Anwendung zur Verringerung eines auf Sonnenbestrahlung zurückzuführenden Erythems.

**2.** Verwendung nach Anspruch 1, dadurch gekennzeichnet, daß das NAD und/oder NADP in einer in Vesikeln mit lamellarer Lipidstruktur eingekapselten wäßrigen Lösung vorliegt (oder vorliegen).

**3.** Verwendung nach Anspruch 2, dadurch gekennzeichnet, daß die Vesikel durch Lipidblättchen definiert sind, die ausgehend von nicht-ionischen Lipiden erhalten wurden.

**4.** Verwendung nach einem der Ansprüche 1 bis 3, dadurch gekennzeichnet, daß man eine in Vesikel aus nicht-ionischen Lipiden eingekapselte wäßrige NAD-Lösung verwendet.

**5.** Verwendung nach Anspruch 2, dadurch gekennzeichnet, daß die Vesikel durch Lipidblättchen definiert sind, welche ausgehend von ionischen Lipiden erhalten wurden.

**6.** Verwendung nach Anspruch 1, dadurch gekennzeichnet, daß das NAD und/oder NADP in einer wäßrigen, nicht-eingekapselten Lösung vorliegt (oder vorliegen).

**7.** Verwendung nach Anspruch 1, dadurch gekennzeichnet, daß das NAD und/oder NADP in einer Emulsion vorliegt (oder vorliegen).

**8.** Verwendung nach einem der Ansprüche 1 bis 7, dadurch gekennzeichnet, daß das NAD und/oder NADP auf die Haut vor Bestrahlung derselben aufgetragen wird (oder werden).

**9.** Verwendung nach einem der Ansprüche 1 bis 7, dadurch gekennzeichnet, daß das NAD und/oder NADP auf die Haut nach Bestrahlung derselben aufgetragen wird (oder werden).

**10.** Verwendung nach einem der Ansprüche 1 bis 9, dadurch gekennzeichnet, daß das NAD und/oder NADP auf die Haut in einer Menge von 10 bis 20 mg/cm$^2$ aufgetragen wird (oder werden).

**11.** Verwendung nach einem der Ansprüche 1 bis 10, dadurch gekennzeichnet, daß das NAD und/oder NADP auf die Haut durch Verteilen einer Zusammensetzung aufgetragen wird (oder werden), welche 0,05 bis 1 Gew.-% NAD oder NADP, bezogen auf das Gesamtgewicht der Zusammensetzung, enthält.

**12.** Kosmetische Zusammensetzung zur Durchführung der Verwendung nach einem der Ansprüche 1 bis 11, dadurch gekennzeichnet, daß sie in einem kosmetisch akzeptablen Träger 0,05 bis 1 Gew.-% NAD und/oder NADP, bezogen auf das Gesamtgewicht der Zusammensetzung, enthält.

**13.** Zusammensetzung nach Anspruch 12, dadurch gekennzeichnet, daS das NAD und/oder NADP in einer in Vesikeln mit lamellarer Lipidstruktur eingekapselten wäßrigen Lösung vorliegt (oder vorliegen).

**14.** Zusammensetzung nach Anspruch 13, dadurch gekennzeichnet, daß die Vesikel durch Lipidblättchen definiert sind, welche ausgehend von nicht-ionischen Lipiden erhalten wurden.

**15.** Zusammensetzung nach einem der Ansprüche 12 bis 14, dadurch gekennzeichnet, daß die Zusammensetzung NAD in einer in Vesikeln aus nicht-ionischen Lipiden eingekapselten wäßrigen Lösung enthält.

**16.** Zusammensetzung nach Anspruch 14 oder 15, dadurch gekennzeichnet, daß die nicht-ionischen amphiphilen Lipide ausgewählt sind unter:
(1) geradkettigen oder verzweigten Polyglycerinethern der Formel:

$$R\ O - [C_3H_5(OH)O]_{\bar{n}} - H$$

worin:

- $-C_3H_5(OH)O$ für die folgenden Strukturen einzeln oder in Kombination steht:
$-CH_2CHOHCH_2O-$ ;

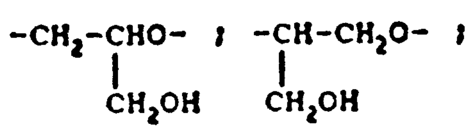

- $\bar{n}$ für einen statistischen Mittelwert von 1 bis 6 steht;
- R bedeutet:
(a) eine geradkettige oder verzweigte, gesättigte oder ungesättigte aliphatiche Kette mit 12 bis 30 Kohlenstoffatomen; oder Kohlenwasserstoffreste von Lanolinalkoholen;
(b) einen Rest $R^1CO$, worin $R^1$ für einen geradkettigen oder verzweigten aliphatischen $C_{11}$-$C_{17}$-Rest steht;

(c) einen Rest $R^2$[$OC_2H_3(R^3)$], worin:

- $R^2$ die für R angegebenen Bedeutungen (a) oder (b) besitzt;
- $OC_2H_3(R^3)$- für die folgenden Strukturen allein oder in Kombination steht:

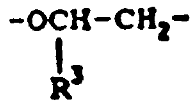 und 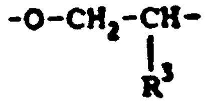

worin $R^3$ die für R angegebenen Bedeutungen (a) besitzt;

(2) geradkettigen oder verzweigten Polyglycerinethern, die zwei Fettketten aufweisen;
(3) polyoxyethylenierten Fettalkoholen und polyoxyethylenierten Sterolen und Phytosterolen;
(4) Polyolethern;
(5) oxyethylenierten oder nicht-oxyethylenierten Polyolestern;
(6) Glycolipiden natürlicher oder synthetischer Herkunft;
(7) Hydroxyamiden der Formel:

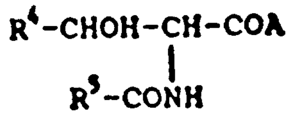

worin:

- $R^4$ einen $C_7$-$C_{21}$-Alkyl- oder -Alkenylrest bedeutet;
- $R^5$ einen gesättigten oder ungesättigten $C_7$-$C_{31}$-Kohlenwasserstoffrest bedeutet;
- COA für eine Gruppe steht, die ausgewählt ist unter folgenden Gruppen:
  - einem Rest

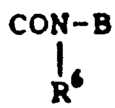

worin:

- B für einen von primären oder sekundären, mono- oder polyhydroxylierten Aminen abgeleiteten Rest steht; und
- $R^6$ ein Wasserstoffatom oder einen Methyl-, Ethyl- oder Hydroxyethylrest bedeutet; und
- einem Rest -COOZ, worin Z den Rest eines $C_3$-$C_7$-Polyols bedeutet.

**17.** Zusammensetzung nach Anspruch 13, dadurch gekennzeichnet, daß die Vesikel durch Lipidblättchen definiert sind, welche ausgehend von ionischen Lipiden erhalten wurden.

**18.** Zusammensetzung nach Anspruch 17, dadurch gekennzeichnet, daß die ionischen amphiphilen Lipide ausgewählt sind unter natürlichen oder synthetischen Phospholipiden und anionischen Verbindungen.

**19.** Zusammensetzung nach einem der Ansprüche 13 bis 18, dadurch gekennzeichnet, daß der (die) Lipidbestandteil(e) der Blättchen der Vesikel mit wenigstens einem Additiv kombiniert ist (sind), das ausgewählt ist unter

- langkettigen Alkoholen und Diolen;
- Sterolen;
- langkettigen Aminen und ihren quaternären Ammoniumderivaten;
- Dihydroxyalkylaminen; polyoxyethylenierten Fettaminen; langkettigen Estern von Aminoalkoholen; und ihren Salzen und quaternären Ammoniumderivaten;
- Phosphorsäureestern von Fettalkoholen;
- Alkylsulfaten;

- bestimmten Polypeptiden und bestimmten Proteinen.

**20.** Zusammensetzung nach Anspruch 12, dadurch gekennzeichnet, daß der Träger dieser Zusammensetzung eine wäßrige Lösung ist.

**21.** Zusammensetzung nach Anspruch 12, dadurch gekennzeichnet, daß der Träger dieser Zusammensetzung eine Emulsion ist.